# EUROPEAN PATENT APPLICATION

(11) **EP 1 371 639 A2**
(43) Date of publication of application: **17.12.2003**
(21) Application number: 03077445.9
(22) Date of filing: 26.10.1998
(51) Int. Cl.: C07C 309/81, C07C 309/20, C08F 28/02, H01M 6/18

(54) **Monomers, ionomers and polymers for electrochemical uses**

(30) Priority: 26.10.1998 US 105662 P
(62) Divisional of application: 99958673.8
(71) Applicant: E.I. DU PONT DE NEMOURS AND COMPANY, Wilmington Delaware 19898 (US)
(72) Inventor: The designation of the inventor has not yet been filed
(74) Representative: Cockerton, Bruce Roger

(57) **Abstract**

A process for forming an ionizable composition, the process comprising: contacting, at a temperature in the range of 0-85°C, a composition comprising the functional group -(CF₂CF₂)ₙ-SO₂F, where n is 1 to 5, with a solution of an alkali metal salt, the pH of said solution not to exceed 13, for a period of time sufficient to obtain the degree of conversion desired to the alkali metal sulfonate form of the polymer.

## Description

This invention pertains to novel fluoro-olefins bearing an ionic functionality or a precursor thereto, a process for the production thereof, and polymers, especially ionomers, formed therefrom, and their application in electrochemical cells, including batteries, fuel cells, electrolysis cells, ion exchange membranes, sensors, electrochemical capacitors, and modified electrodes, most particularly in lithium batteries. The polymers of the invention are useful in the formation of films and coatings with high chemical resistance and good physical properties. The olefinic compositions of the invention are useful as monomer precursors to the polymers and ionomers of the invention; they are also useful, in acid form, as super-acid catalysts for reactions such as Friedel-Crafts alkylation.

### BACKGROUND OF THE INVENTION

CF₂=CFCF₂OCF₂CF₂SO₂F is disclosed in Krespan, U.S. Patent 4,275,225. Also disclosed are copolymers, including terpolymers, therewith with ethylenically unsaturated comonomers, and the ionomers formed therefrom.

Preparation of CF₂ClCFCl(CF₂CF₂)₂I is described in Feiring et al, U.S. Patent 5,350,821.

It has long been known in the art to form ionically conducting membranes and gels from organic polymers containing ionic pendant groups. Such polymers are known as ionomers. Particularly well-known ionomer membranes in widespread commercial use are Nafion® Membranes available from E. I. du Pont de Nemours and Company. Nafion® is formed by copolymerizing tetra-fluoro ethylene (TFE) with perfluoro(3,6-dioxa-4-methyl-7-octenesulfonyl fluoride), as disclosed in U.S. Patent 3,282,875. Also known are copolymers of TFE with perfluoro(3-oxa-4-pentene sulfonyl fluoride), as disclosed in U.S. Patent 4,358,545. The copolymers so formed are converted to the ionomeric form by hydrolysis, typically by exposure to an appropriate aqueous base, as disclosed in U.S. Patent 3,282,875. Lithium, sodium and potassium are all well known in the art as suitable cations for the above cited ionomers.

Formation of ionomers and acid copolymers by hydrolysis of the sulfonyl fluoride functionality in copolymers of TFE and fluoro alkoxy sulfonyl fluorides is well known in the art. The art teaches exposure of the copolymer to strongly basic conditions.

See for example, Ezzell et al. U.S. 4,940,525, wherein is used 25 wt % NaOH(aq) for 16 hours at 80-90°C; Banerjee et al. U.S. 5,672,438, wherein is used 25 wt % NaOH for 16 hours at 90°C, or, in the alternative, an aqueous solution of 6-20% alkali metal hydroxide and 5-40% polar organic liquid (e.g., DMSO) for 5 minutes at 50-100°C; Ezzell et al. U.S. 4.358.545 wherein is used .05N NaOH for 30 minutes for 50°C; Ezzell et al. U.S. 4,330,654, wherein is used 95% boiling ethanol for 30 minutes followed by addition of equal volume of 30% NaOH (aq) with heating continued for 1 hour; Marshall et al. EP 0345964 A1, wherein is used 32 wt % NaOH (aq) and methanol forl6 hours at 70°C, or, in the alternative, an aqueous solution of 11 wt % KOH and 30 wt % DMSO for 1 hour at 90°C; and, Barnes et al. U.S. 5,595.676, wherein is used 20 wt % NaOH (aq) for 17 hours at 90°C.

Doyle et al. (WO98/20573) disclose a highly fluorinated lithium ion exchange polymer electrolyte membrane (FLIEPEM) exhibiting a conductivity of at least 0.1 mS/cm comprising a highly fluorinated lithium ion exchange polymer membrane (FLIEPM), the polymer having pendant fluoroalkoxy lithium sulfonate groups, and wherein the polymer is either completely or partially cation exchanged; and, at least one aprotic solvent imbibed in said membrane. Electrodes and lithium cells are also disclosed.

In the polymers above-cited, the fluorine atoms provide more than one benefit. The fluorine groups on the carbons proximate to the sulfonyl group in the pendant side chain provide the electronegativity to render the cation sufficiently labile so as to provide high ionic conductivity. Replacement of those fluorine atoms with hydrogen results in a considerable reduction in ionic mobility and consequent loss of conductivity.

The remainder of the fluorine atoms, such as those in the polymer backbone, afford the chemical and thermal stability to the polymer normally associated with fluorinated polymers. This has proven to be of considerable value in such applications as the well-known "chlor-alkali" process. However, highly fluorinated polymers also have disadvantages where there is less need for high chemical and thermal stability. The fluorinated monomers are more expensive than their olefin counterparts, require higher processing temperatures, and often require expensive corrosion resistant processing equipment. Furthermore, it is difficult to form solutions and dispersions of fluoropolymers. Additionally, it is difficult to form strong adhesive bonds with fluoropolymers. In materials employed in electrochemical cells, for example, it may be advantageous to have better processibility at some cost to chemical and thermal stability. Thus, there is an incentive to develop ionomers with highly labile cations having reduced fluorine content.

Numerous publications disclose polyethers with either proximal ionic species in the polymer or in combination with ionic salts. Conductivities are in the range of 10⁻⁵ S/cm and less. Le Nest et al., Polymer Communications 28, 303 (1987) disclose a composition of polyether glycol oligomers joined by phosphate or thiophosphate moieties hydrolyzed to the related lithium ionomer. In combination with propylene carbonate, conductivity in the range of 1-10 x10⁻⁴ S/cm was realized. A review of the related art is found in Fauteux et al., Electrochimica Acta 40, 2185 (1995)

Benrabah et al, Electrochimica Acta. 40. 2259 (1995) disclose polyethers crosslinked by lithium oxytetrafluorosulfonates and derivatives. No aprotic solvents are incorporated. With the addition of lithium salts conductivity of < 10⁻⁴ S/cm was achieved.

Armand et al., United States Patent 5,627,292 disclose copolymers formed from vinyl fluoroethoxy sulfonyl fluorides or cyclic ethers having fluoroethoxy sulfonyl fluoride groups with polyethylene oxide, acrylonitrile, pyridine and other monomers. Lithium sulfonate ionomers are formed. No aprotic solvents are incorporated. Conductivity was <10⁻⁴ S/cm.

Narang et al., United States Patent 5,633,098 disclose polyacrylate copolymers having a functionalized polyolefin backbone and pendant groups containing tetrafluoroethoxy lithium sulfonate groups. The comonomers containing the sulfonate groups are present in molar ratios of 50-100%. Compositions are disclosed comprising the polymer and a solvent mixture consisting of propylene carbonate, ethylene carbonate, and dimethoxyethane ethyl ether. Ionic conductivity of those compositions was in the range of 10⁻⁴-10⁻³ S/cm.

In the present invention, both fully and partially fluorinated species are disclosed.

### SUMMARY OF THE INVENTION

This invention provides for an olefinic composition represented by the formula:

CF₂=CF(CF₂CF₂)ₙSO₂F **1**

where n= 1 to 5.

Further provided is an ionizable olefinic composition represented by the formula

CF₂=CF(CF₂CF₂)ₙSO₃M

wherein n = 1 to 5 and M is a univalent metal.

Further provided is a process for synthesizing **1**, the process comprising
Reacting CF₂ClCFCl(CF₂)ₙI, wherein n =1-5 with Na₂S₂O₄ and NaHCO₃ in a mixture of water and an aprotic polar solvent at a temperature in the range of room temperature to 110°C, to form CF₂CICFCI(CF₂)ₙSO₂Na;
Reacting the said CF₂CICFCI(CF₂)ₙSO₂Na with Cl₂ in water or a mixture of water and an aprotic polar solvent at a temperature in the range of 0-100°C to form CF₂CICFCI(CF₂)ₙSO₂CI;
Reacting the said CF₂CICFCI(CF₂)ₙSO₂CI with a univalent metal fluoride in an anhydrous aprotic polar solvent to form CF₂CICFCI(CF₂)ₙSO₂F;
Reacting said CF₂CICFCI(CF₂)ₙSO₂F with Zn in an alcohol or a mixture of acetic acid and alcohol to form CF₂=CF(CF₂)ₙSO₂F; and,
separating the product.

Further provided are polymers, including ionomers, comprising 0.1 to 50 mol-% of monomer units represented by the formula 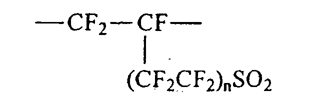 wherein n = 1 to 5, X is F or -OM where M is a univalent metal or hydrogen.

Further provided is an ionomer comprising monomer units of vinylidene fluoride and 0.1 to 50 mol-% of monomer units represented by the formula 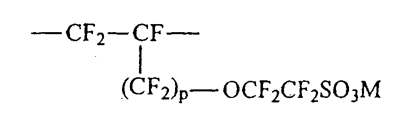 wherein p is 1 to 10 and M is a univalent metal.

The present invention further provides for a process for forming an ionizable composition most suitable for use with base-sensitive compositions such as those herein disclosed. The process comprises contacting at a temperature in the range of 0-85°C a composition comprising the functional group -(CF₂CF₂)ₙ-SO₂F where n is 1 to 5 with a solution of an alkali metal salt, the pH of said solution not to exceed 13, for a period of time sufficient to obtain the degree of conversion desired to the alkali metal sulfonate form of the polymer.

The present invention further provides for an ionically conductive composition comprising an ionomer of the invention and a liquid imbibed therewithin.

The present invention further provides for an ionically conductive composition comprising the ionizable olefinic composition of the invention and a liquid

The present invention further provides for an electrode comprising at least one electrode active material and the ionomer of the invention.

The present invention further provides for an electrochemical cell comprising a positive electrode. a negative electrode, a separator disposed between the positive and negative electrodes. and a means for connecting the cell to an outside load or source wherein at least one of the group consisting of the separator, the cathode, and the anode, comprises the ionomer of the invention.

### DETAILED DESCRIPTION OF THE INVENTION

This invention provides for an olefinic composition represented by the formula:

CF₂=CF(CF₂CF₂)ₙSO₂F **1**

where n is 1 to 5, preferably n is 1. The olefinic composition **1** is preferably employed as a monomer in a free-radical polymerization process.

The process for synthesizing **1** comprises a first step of reacting CF₂CICFCI(CF₂CF₂)ₙI with Na₂S₂O₄ and NaHCO₃ in a mixture of water and an aprotic polar solvent at a temperature in the range of room temperature to 110°C, to form CF₂CICFCI(CF₂CF₂)ₙSO₂Na. n is 1 to 5, preferably n is 1. The aprotic polar solvent is preferably selected from the group consisting of acetonitrile, dimethylformamide (DMF), tetrahydrofuran (THF), dimethylsulfoxide (DMSO), dimethylacetamide (DMAC). Preferably the temperature is in the range of room temperature to 50°C.

The so produced CF₂ClCFCl(CF₂CF₂)ₙSO₂Na is then reacted with Cl₂ in water or a mixture of water and an aprotic polar solvent at a temperature in the range of 0-200°C, preferably 5-100°C, most preferably 25°C-100°C, to form CF₂CICFCI(CF₂CF₂)ₙSO₂CI. The aprotic polar solvent is preferably selected from the group hereinabove cited.

The CF₂CICFCI(CF₂CF₂)ₙSO₂CI is then reacted with a univalent metal fluoride in an anhydrous aprotic polar solvent at a temperature of 0-100°C, preferably 20-50°C to form CF₂CICFCI(CF₂CF₂)ₙSO₂F. Preferably the univalent metal is an alkali metal, most preferably, potassium. Preferably the aprotic polar solvent is selected from the group hereinabove cited.

The CF₂CICFCI(CF₂CF₂)ₙSO₂F is reacted with Zn in alcohol or a mixture of acetic acid and alcohol at a temperature in the range of 30-150°C, preferably 50°C to 100°C to form CF₂=CF(CF₂CF₂)ₙSO₂F. The alcohol is preferably ethanol or isopropanol. Most preferably the solvent is a mixture of acetic acid and isopropanol. The final sulfonyl fluoride product may be separated by any convenient method known in the art. such as by distillation or recrystallization.

The olefinic composition **1** may be copolymerized according to means known in the art to polymers having 0.1 to 50 mol-% of monomer units represented by the formula 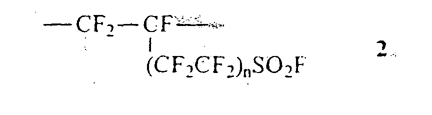 wherein n= 1 to 5. The polymer 2 is preferably employed as a thermoplastic precursor to an ionomer, but may also be employed as a coating or film-forming resin. Krespan, US 4,275, 225 teaches the formation of polymers and copolymers from a monomer represented by the formula

CF₂=CF(CF₂CF₂)ₙ - OCF₂CF₂SO₂F **3**

the teachings encompassing ethylenically unsaturated comonomers, with TFE preferred. The teachings of Krespan may be extended to the copolymerization of the olefinic composition **1** of the present invention to form the polymer of the present invention.

Monomers suitable for use in forming the copolymers of the invention include any ethylenically unsaturated compound which is copolymerizable by a free radical polymerization process. Preferred monomers include ethylene, propylene, tetrafluoroethylene (TFE), hexafluoropropylene (HFP), chlorotrifluoroethylene (CTFE), trifluoroethylene, vinylidene fluoride (VF₂), and vinyl fluoride. Most preferred are ethylene and VF₂.

Mixtures of monomers are also suitable for the formation of terpolymers of the invention. Preferred mixtures of monomers include VF₂ and HFP, VF₂ and CTFE, TFE and ethylene, VF₂ and TFE, and VF₂ and trifluoroethylene. Most preferred mixtures are VF2/HFP, TFE/E, CTFE/E.

The exact degree of incorporation of co-monomers in the polymer will depend upon relative reactivities of the respective monomers under the conditions of reaction. The polymers of the invention are primarily random copolymers having 0.1-ca. 50 mol-%, preferably 1-20 mol-%, most preferably 3-12 mol-%, of the sulfonyl-containing monomer unit incorporated therein. Polymers containing-some degree of block copolymer character are encompassed by the present invention but are less preferred.

It is desirable for the purposes of the present invention, namely the formation of components useful in lithium batteries, to incorporate into the polymer ca. 3-12 mol-% of the ionizable monomer units or their precursors. When TFE is employed as a comonomer with the monomer of the invention, **1** the degree of incorporation of **1** tends to be less than about 3 mol-%. A means for improving the degree of incorporation is to employ VF₂ as a comonomer in place of TFE. This results in the ready incorporation of ca. 3-12 mol-% of **1**.

Copolymers of **1** with VF₂ tend to exhibit excessive crystallinity, particularly at the lower concentration end of **1**. High crytallinity is undesirable because it negatively affects conductivity and toughness. Thus incorporation of a crystallinity disrupting termonomer, such as HFP, is desirable.

Alternatively, a similar balance between incorporation and crystallinity may be achieved by producing a terpolymer of **1** with ethylene and TFE.

Polymerization can be conducted by block polymerization, solution polymerization, suspension polymerization, and emulsion polymerization. Bis(perfluoropropionyl)peroxide and bis(4-t-butylcyclohexylperoxy) dicarbonate were found to be suitable free-radical initiators for suspension polymerization or solution polymerizations. In aqueous polymerization, inorganic peroxides such as ammonium or potassium persulfate have been found suitable. In aqueous polymerization it is preferred to employ surfactants such as perfluorocarboxylic salts, with C₇F₁₅CO₂NH₄ most preferred. Other initiators such as are known in the art are also suitable for use.

The copolymers of the present invention, having monomer units represented by the formula **2** can for the most part be hydrolyzed to form the ionomers of the present invention by methods known in the art. However, in the preferred embodiments wherein VF₂ is present in amounts of 10 mol-% or greater, the methods taught in the art are not operable.

In one embodiment, ionomers of the present invention comprise 0.1 to 50 mol-% of monomer units represented by the formula: 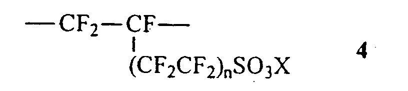 wherein n =1 to 5 and X is hydrogen or a univalent metal. Preferably n=1 and X is Li.

The preferred ionomers of the invention comprise monomer units of VF₂ and 0.1 to 50 mol-% of monomer units represented by the formula 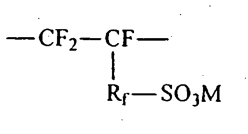 wherein R_{f} is represented by the formula

- (CF₂)ₚ - (OCF₂CF₂)ₘ-

where p is 1 to 10 and m = 0 or 1, with the proviso that when m = 0, p is an even number. Preferably p is 1, and m = 1. M is a univalent metal or hydrogen; preferably M is an alkali metal; most preferably M is Li. When m = 1, the monomer 3 is synthesized according to the teachings of Krespan op.cit.

The methods taught in the art for hydrolyzing sulfonyl fluoride to a sulfonate salt involve the use of strong bases at temperatures well above room temperature. Such methods are highly effective when applied to polymers having chemically inert backbones. However, the methods of the art applied to less stable species result in extensive degradation.

It is known in the art that VF₂ homopolymers and copolymers are subject. to attack by strong bases such as the alkali metal hydroxides taught in the hydrolysis procedures of the art, see W.W. Schmiegel in Die Angewandte Makromolekulare Chemie, 76/77 pp 39ff, 1979. The sensitivity to base attack of the VF₂ copolymer formed in the practice of the present invention has prevented the development of a single-ion conducting ionomer based upon VF₂. There simply is no means taught in the art for making the ionomer.

The present invention further provides for a process for forming an ionizable composition most suitable for use with base-sensitive compositions such as those herein disclosed. The process comprises contacting at a temperature in the range of 0-85°C a composition comprising the functional group -(CF₂CF₂)ₙ-SO₂F where n = 1 to 5 with a solution of an alkali metal salt or hydroxide, the pH of said solution not to exceed 13, for a period of time sufficient to obtain the degree of conversion desired to the alkali metal sulfonate form of the polymer.

Base-unstable species suitable for use in the hydrolysis process of the invention include species comprising -(CF₂CF₂)ₙ-SO₂F where n = 1 to 5 and unsaturated carbon-carbon bonds, such as **1** or **3**, and sulfonyl fluoride-containing copolymers comprising vinylidene fluoride, including copolymers or terpolymers of **1** or **3** with vinylidene fluoride. It is a surprising result of the hydrolysis process of the invention that conditions exist wherein the desired hydrolysis may be accomplished in the absence of the undesired side reactions characteristic of the harsher conditions employed in the art.

It will be understood by one of skill in the art that there are different degrees of base-instability in the base-sensitive species preferred for the practice of the hydrolysis process of the invention. For example, a vinyl ether copolymer with vinylidene tluoride is more susceptible to attack by base than is a vinylidene fluoride copolymer not containing vinyl ether.

One of skill in the art will appreciate that hydrolysis cannot proceed unless there is contact between the hydrolyzing solution and the hydrolyzable species. Such contact may be achieved in solution in a common solvent, by finely subdividing a solid, such as a polymeric, hydrolyzable species, or by causing a hydrolyzable polymer to swell by employing solvents for the hydrolyzing solution which are also soluble in the polymer. Generally preferred are the mildest hydrolysis conditions possible consistent with timely conversion of the sulfonyl fluoride into the ionic form desired. The degree of conversion can be conveniently monitored by the disappearance of the characteristic infrared absorption band for the sulfonyl fluoride group at about 1470 cm⁻¹. Alternatively, ¹⁹F NMR spectroscopy may be used as described in the examples.

A preferred hydrolysis process of the invention comprises contacting the sulfonyl fluoride-containing monomer or polymer with a mixture of alkali metal carbonate and methanol at a temperature in the range of room temperature to 65°C for a sufficient length of time to convert the desired percentage of sulfonyl fluorides to the related metal sulfonate. The alkali metal carbonate is selected to provide the cation desired for the intended application. Suitable alkali metal carbonates include Li₂CO₃, Na₂CO₃, and K₂CO₃, with Li₂CO₃ most preferred.

Other cationic forms of the ion-exchange membrane can be acheived using ion-exchange procedures commonly known in the art (see for example Ion Exchange by F. Helfferich, McGraw Hill, New York 1962). For example, the protonic form of the membrane is preferably obtained by immersing the alkali metal-ionomer into an aqueous acid.

Silver and copper sulfonate ionomers can be made by ion exchange with the alkali metal sulfonate form of the polymer. For example, repeated treatment of the lithium sulfonate ionomer with an aqueous solution of a silver salt such as silver fluoride or silver perchlorate would produce at least a partially cation exchanged silver sulfonate ionomer. In a similar fashion, the cuprous sulfonate ionomer can be produced by repeated treatment of the alkali metal sulfonate ionomer with an aqueous acidic solution of a copper salt such as cuprous chloride.

In many applications, the ionomer is preferably formed into a film or sheet. Films of the ionomer may be formed according to processes known in the art. In one embodiment, the thermoplastic sulfonyl fluoride-containing polymer is extrusion melt cast onto a cooled surface such as a rotating drum or roll, whence it is subject to hydrolysis according to the process hereinabove described.

In a second embodiment, the sulfonyl fluoride-containing copolymer is dissolved in a solvent, the solution cast onto a smooth surface such as a glass plate using a doctor knife or other device known in the art to assist in depositing films on a substrate, and the resultant film subject to hydrolysis.

In a third embodiment, the sulfonyl fluoride-containing copolymer- resin is subject to hydrolysis by dissolution or suspension in a hydrolyzing medium. followed by optional addition of cosolvent, and filtration or centifugation of the resulting mixture, and finally solvent casting of the ionomer solution onto a substrate using a doctor knife or other device known in the art to assist in depositing films on a substrate.

The sulfonyl-fluoride-containing copolymer may exhibit a tendency to dissolve during hydrolysis when the concentration of the sulfonyl fluoride moiety exceeds about 5 mol-%. Thus for the purpose of achieving better control over the film forming process, it is found preferable to suspend the non-ionic sulfonyl fluoride-containing precursor polymer in a solvent or combination of solvents such as, methanol, dimethyl carbonate, or mixtures thereof, also containing the hydrolyzing agent, preferably Li₂CO₃ thereby hydrolyzing the polymer in solution. The thus hydrolyzed polymer is then cast as a film from solution.

The ionomer of the present invention, however formed, exhibits a low-level of ionic conductivity in the dry state, at room temperature, typically ca. 10⁻⁶ S/cm. It may be combined with a liquid to achieve higher levels of ionic conductivity. Depending upon the requirements of the application, the ionomer will be in the acid form or the metal salt form, the particular metal being determined by the application as well. The liquid employed therewith will likewise be dictated by the application. In general terms, it has been found in the practice of the invention that conductivity of the liquid-containing ionomer increases with increasing % weight uptake, increasing dielectric constant, and increasing lewis basicity of the liquid; while conductivity has been observed to decrease with increasing viscosity and increasing molecular size of the liquid employed. Of course, other considerations come into play as well. For example, excessive solubility of the ionomer in the liquid may be undesirable. Or, the liquid may be electrochemically unstable in the intended use.

In a preferred embodiment of the present invention, the lithium ionomer is combined with an aprotic solvent to form a conductive composition suitable for use in lithium batteries. Preferred aprotic solvents include dimethylsulfoxide, ethylene carbonate, propylene carbonate, dimethoxyethane, gamma-butyrolactone, mixtures thereof, and mixtures thereof with dimethyl carbonate. Most preferred is a mixture of ethylene carbonate and dimethyl carbonate.

One particularly preferred embodiment comprises the lithium ionomer comprising monomer units of VF₂ combined with aprotic solvents, preferably organic carbonates. It is in lithium batteries that the particularly useful attributes of the ionomer of the invention are particularly noteworthy. High solvent uptake characteristic of VF₂ polymers results in desirably high ionic conductivity in the solvent-swollen membrane. Furthermore the VF₂ imparts highly desirable electrochemical stability in the lithium battery environment.

It is found in the practice of the invention that an ionomer of the invention containing at least 50% VF₂, more preferably at least 80% VF₂, may become excessively plasticized by the solvents imbibed within it, with concomitant loss of the physical integrity of the membrane. In some applications, it may be desirable to enhance the properties of the solvent-swollen membrane. Means available for improving the mechanical properties include: 1) Incorporation into the polymer by means known in the art, and following the synthetic pathway hereinbelow described, a non-ionic third monomer that is less solvent sensitive; 2) formation by known means of a polymer blend with a non-ionic polymer that is less solvent sensitive; 3) blending by known means of the ionomer of the invention with an inert filler; 4) blending different compositions of ionic copolymers; and 5) crosslinking.

Suitable third monomers include tetrafluoroethylene, chlorotrifluoroethylene, ethylene, hexafluoropropylene, trifluoroethylene, vinyl fluoride, vinyl chloride, vinylidene chloride, perfluoroalkylvinyl ethers of the formula CF₂=CFOR_{f} where R_{f} = CF₃, C₂F₅ or C₃F₆. Preferred termonomers include tetrafluoroethylene, hexafluoropropylene, ethylene and the perfluoroalkylvinyl ethers. Termonomers are preferably present in the polymer at a concentration of up to 30 mol %.

Polymers suitable for blending with the preferred ionomers of the invention include poly(tetrafluoroethylene) and copolymers thereof with hexafluoropropylene or perfluoroalkyl vinyl ethers, polyvinylidene fluoride homopolymer and a copolymer thereof with hexafluoropropylene, polymethylmethacrylate, polyethylene oxide, and poly(vinyl chloride). A preferred composition comprises 25 to 50 weight % PVF₂ homopolymer blended with the preferred ionomer of the present invention. These materials are easily blended together by means common in the art such as dissolution and mixing in a common solvent such as acetone and then casting a membrane.

Suitable inert fillers include SiO₂, Al₂O₃, TiO₂, or CaF₂. Small and high surface area particles less than 1.0 micron in diameter are desired, such as are available for the preferred grade of SiO₂ under the trade name Cab-o-sil® TS-530 silica. Loadings of up to 50 weight % filler are preferred.

The relatively high solubility of the preferred ionomers of the present invention and their sulfonyl fluoride precursors provides a benefit in ease of processing during fabrication of the components of a battery but may be problematical during final assembly of the desired battery product. In a preferred embodiment of the battery of the present invention, a battery is formed from one or more electrochemical cells formed by laminating together in film form the anode, cathode, and separator compositions of the present invention, all of which have been rigorously dried prior to addition of a liquid selected from the group of organic carbonates and mixtures thereof, a mixture of ethylene carbonate and dimethyl carbonate being most preferred. Organic carbonates will not only swell the ionomeric polymer, but may also dissolve the polymer depending on the composition thereof, the primary determining factor being the degree of crystallinity, which in turn is related to the concentration of ionic comonomer in the polymer. The challenge is to swell the ionomer with solvent while minimizing dissolution of the polymer.

One way to achieve the necessary balance is to use the methods hereinabove described for improving the physical integrity of the solvent-containing ionomer. Another approach comprises dissolution of the ionomer into the preferred organic carbonate solvents, followed by introduction of the resulting solution into the pores of an inert, porous polymer support such as Celgard® porous polypropylene, available from Hoechst-Celanese, or Gore-Tex microporous PTFE, available from W.L. Gore Associates, Newark, DE.

The preferred electrode of the invention comprises a mixture of one or more electrode active materials in particulate form, the ionomer of the invention, at least one electron conductive additive, and at least one organic carbonate. Examples of useful anode active materials include, but are not limited to, carbon (graphitic, coke-type, mesocarbons, polyacenes, and the like) and lithium-intercalated carbon, lithium metal nitrides such as Li_{2.6}Co_{0.4}N, tin oxides, lithium metal, and lithium alloys, such as alloys of lithium with aluminum, tin, magnesium, mercury, manganese, iron, and zinc. Lithium intercalation anodes employing carbon are preferred. Useful cathode active materials include, but are not limited to, transition metal oxides and sulfides, lithiated transition metal oxides and sulfides, and organosulfur compounds. Examples of such are cobalt oxides, manganese oxides, molybdenum oxides, vanadium oxides, sulfides of titanium, molybdenum and niobium, lithiated oxides such as spinel lithium manganese oxides Li₁₊ₓMn₂₋ₓO₄, chromium-doped spinel lithium manganese oxides LiₓCr_{y}Mn_{z}O₄, LiCoO₂, LiNiO₂, LiNiₓCo₁₋ₓO₂ where x is 0 <x< 1, with a preferred range of 0.5 < x < 0.95, LiCoVO₄, and mixtures thereof. LiNiₓCo₁₋ₓO₂ is preferred. A highly preferred electron conductive aid is carbon black, preferably Super P carbon black, available from the MMM S.A. Carbon, Brussels, Belgium, in the concentration range of 1-10%. Preferably, the volume fraction of the lithium ionomer in the finished electrode is between 4 and 40%.

The electrode of the invention may conveniently be made by dissolution of all polymeric components into a common solvent and mixing together with the carbon black particles and electrode active particles. For cathodes the preferred electrode active material is LiNiₓCo₁₋ₓO₂ wherein 0 < x < 1, while for anodes the preferred electrode active material is graphitized mesocarbon microbeads. For example, a preferred lithium battery electrode of the invention can be fabricated by dissolving ionomer of the invention in a mixture of acetone and dimethylformamide, followed by addition of particles of electrode active material and carbon black, followed by deposition of a film on a substrate and drying. The resultant preferred electrode will comprise electrode active material, conductive carbon black, and ionomer of the invention, where, preferably, the weight ratio of ionomer to electrode active material is between 0.05 and 0.8 and the weight ratio of carbon black to electrode active material is between 0.01 and 0.2. Most preferably the weight ratio of ionomer to electrode active material is between 0.1 and 0.25 and the weight ratio of carbon black to electrode active material is between 0.02 and 0.1. This electrode can then be cast from solution onto a suitable support such as a glass plate or current collector metal foil, and formed into a film using techniques well-known in the art. The electrode film thus produced can then be incorporated into a multi-layer electrochemical cell structure by lamination, as hereinbelow described.

It may be desirable to incorporate into the electrode composition of the invention additional polymers or solvents for such purposes as improving the binding of the components thereof, or providing improved structural integrity of an article fabricated therefrom. One particularly preferred additional material is polyvinylidene fluoride homopolymer, which may be incorporated simply by dissolving the polymer into the same solution from which the electrode is being formed, as hereinabove described.

In an alternative process, the dispersion of electrode-active material and optional carbon black and other adjuvants can first be cast onto a surface followed by addition of the ionomer of the invention in organic carbonate solution.

The invention is further described in the following specific embodiments.

### EXAMPLES

### EXAMPLE 1

To a stirred solution at room temperature of 157 g of Na₂S₂O₄, 75.6 g of NaHCO₃ in 400 mL of acetonitrile and 400 mL of water was dropwise added 227.6 g of CF₂CICFCICF₂CF₂I, which was synthesized according to the teachings of Feiring et al, U.S. Patent 5,350,821. After the addition was complete, the resulting mixture was stirred overnight. Solids were removed by filtration and washed with ethyl acetate. The filtrate was extracted with ethyl acetate three times The combined organic layers were washed with aqueous NaCl solution and evaporated to give 200 g of crude salt. which was dissolved in 700 mL of water and treated with chlorine at 5°C over two hours. Clear yellow organic layer was separated and washed with aqueous NaCI solution and dried over MgSO₄. After removal of MgSO₄, organic layer was distilled to give CF₂CICFCICF₂CF₂SO₂CI product 146.3 g.

A mixture of 135 g of the thus produced CF₂CICFCICF₂CF₂SO₂CI and 40 g of KF in 200 mL of acetonitrile was stirred at room temperature for 24 hours and at 50°C for 6 hours. The mixture was poured into water and the lower layer was separated and washed with water and aqueous NaCI solution to give 117.5 g of 99.5% pure CF₂CICFCICF₂CF₂SO₂F product.

To a stirred suspension of 30 g of Zn and 35 mL of isopropyl alcohol and 45 mL of acetic acid was slowly added 77 g of the thus produced CF₂CICFCICF₂CF₂SO₂F at 90°C. After the addition was complete, the reaction mixture was stirred for 8 hours. Volatiles were distilled out and the distillate was poured into water. The lower layer was separated, washed with NaHCO₃ solution and water, and distilled to give 27.3 g of CF₂=CFCF₂CF₂SO₂F, bp 72°C. ¹⁹F NMR: +45.1 (s, 1F), -85.0 (dd, J = 45.2 hz, J = 37.7 Hz, 1F), -102.7 (dd, J = 45.2 Hz, J = 124.3 Hz, 1F), -110.4 (d, J = 3.8 Hz, 2F), -116..0 (m, 2F), -190.0 (ddt, J = 124.3 Hz, J = 22.6 Hz, J = 3.8 Hz, 1F).

### EXAMPLE 2

Following the teachings of Krespan, U.S. Patent 4,275,225, to a stirred suspension of 37.7 g of KF and 500 mL of TG was rapidly added 108 g of FCOCF₂SO₂F at 0°C and the resulting mixture was stirred at 0°C for 20 min. 150 g of CF₂=CFCF₂OSO₂F was slowly added to the solution via an addition funnel at -5 to 0°C over 1.5 hours. After the addition was complete, the mixture was stirred at -5°C for 1 hour, and then warmed to room temperature over 2 hours. Volatiles were transferred into a -78°C trap under vacuum. 188.5 g of 97% pure CF₂=CFCF₂OCF₂CF₂SO₂F (E77787-114) was obtained. ¹⁹F NMR: +45.1 (pent, J = 6.0 Hz, 1F), -71.5 (m, 2F), -82.6 (m, 2F), -89.9 (ddt, J = 54.3 Hz, J = 39 Hz, J = 7.4 Hz, 1F), -103.7 (ddt, J = 117.9 Hz, J =54.3 Hz, J=25.3 Hz, 1F), -112.5 (t, J = 2.6 Hz, 2F), -190.7 (ddt, J = 117. Hz, J = 39.0 Hz, J= 14 Hz, 1F).

A 240-mL Shaker tube was charged with 100 mL of 1,1,2-trichlorotrifluoroethane (F113), 12 g of thus produced CF₂=CFCF₂OCF₂CF₂SO₂F and 0.34 g of Perkadox™ bis(4-t-butylcyclohexylperoxy) dicarbonate obtained from Pennwalt Chemical Corp, now defunct. The reaction vessel was cooled in dry ice and degassed and replaced with nitrogen gas repeatedly. 36 g of vinylidene fluoride was added into the vessel and the tube was heated at 50°C for 10 hours and 80°C for 2 hours. After completion of the polymerization, the unreacted VF₂ was removed and white solid was dissolved in 500 mL of acetone. The acetone solution was slowly poured into MeOH. The solid was filtered and washed with MeOH and dried in a partial vacuum oven at 80°C to give 38.2 g of polymer. IR(KBr): 1463 cm⁻¹(SO₂F). 19F NMR indicated about 5 mol % of CF₂=CFCF₂OCF₂CF₂SO₂F Elementary analysis indicated 3.3 mol % of CF₂=CFCF₂OCF₂CF₂SO₂F based on C, 34.7% and H, 2.70%. Mw = 4.1X10⁴ and Mn = 8.6x10³. DSC showed that the polymer had Tm 146°C. Decomposition temperature was 400°C by TGA in N₂.

A suspension solution of 3.0 g of the copolymer so made and 1.0 g of Li₂CO₃ in 15 mL of water and 15 mL of MeOH was stirred for 2 days and then diluted with 100 mL of water. The solid was filtered and washed with water and dried in an oven at 80°C to give 2.67 g of white polymer which was soluble in DMAc. ¹⁹F NMR indicated no sulfonyl fluoride. The polymer could be pressed into a thin film. TGA indicated that decomposition temperature was 250°C and DSC showed Tm at 140°C.

### EXAMPLE 3

A 240-mL Shaker tube was charged with 100 mL of 1,1,2-trichlorotrifluoroethane (F113), 12 g of the CF₂=CFCF₂OCF₂CF₂SO₂F made in Example 2 and 0.24 g of Perkadox™ bis(4-t-butylcyclohexylperoxy) dicarboylate. The reaction vessel was cooled in dry ice and degassed and replaced with nitrogen gas repeatedly. 18 g of vinylidene fluoride was added into the vessel and the tube was heated at 50°C for 10 hours and 80°C for 2 hours. After completion of the polymerization; the unreacted VF₂ was removed and white solid was dissolved in 400 mL of acetone. The acetone solution was slowly poured into a 1:1 MeOH and water solution. The solid was filtered and washed with MeOH and dried in a partial vacuum oven at 80°C to give 16.2 g of polymer. IR(KBr): 1460 cm⁻¹ (SO₂F). Elementary analysis indicated 7.3 mol % of CF₂=CFCF₂OCF₂CF₂SO₂F based on C, 32.1% and H, 2.14% and ¹⁹F NMR (CD₃COCD₃) indicated 8.4 mol % of CF₂=CFCF₂OCF₂CF₂SO₂F: +45.7 (s, SO₂F), -76.5 (d, J = 147 Hz), -78.1 (d, J = 147 Hz),-81.6 (s), -91 (m), -111.6 (s), -110 to -117(m), -183.6). Mw = 8.8X10⁴ and Mn = 2.7X10⁴. DSC showed that the polymer had Tm 123°C. Decomposition temperature was 400°C by TGA in N₂.

A mixture of 2.8 g of the polymer so made. 10 mL of saturated LiOH in 50 mL of water and 50 mL of MeOH was stirred at room temperature for two days. After being diluted with 200 mL of water, the mixture was filtered and washed with water 5 times and dried at 70°C in a partial vacuum oven to give ionic polymer 2.7 g. ¹⁹F NMR indicated complete conversion of the sulfonyl fluoride group into lithium salt of sulfonic acid.

### EXAMPLE 4

A 240-mL Shaker tube was charged with 100 mL of 1.1.2-trichlorotrifluoroethane (F113). 12 g of the CF₂=CFCF₂OCF₂CF₂SO₂F of Example 2 and 0.34 g of Perkadox™. The reaction vessel was cooled in dry ice and degassed and replaced with nitrogen gas repeatedly. 36 g of vinylidene fluoride and 7 g of HFP were added into the vessel and then the tube was heated at 50°C for 10 hours and 80°C for 2 hours. After completion of the polymerization. the unreacted VF₂ and HFP were removed and white solid was dissolved in 400 mL of acetone. The acetone solution was slowly poured into a stirred 800 mL of MeOH. Solid was filtered and washed with MeOH and dried in a partial vacuum oven at 80°C to give 35.5 g of polymer. IR(KBr) 1460 (SO₂F). Elementary analysis indicated 2.6 mol % of CF₂=CFCF₂OCF₂CF₂SO₂F and 8 mol % of HFP based on C, 33.30% and H, 2.30%. GPC showed Mw = 1.3X10⁵ and Mn = 4.9X10⁴. DSC showed that the polymer had a broad Tm, 140°C. Decomposition temperature was 400°C by TGA in N₂.

A suspension of 10.6 g of the polymer so produced, 2.3 g of Li₂CO₃ in 70 mL of MeOH and 2 mL of H₂O was stirred at room temperature for 5 hours and at 50°C for 24 hours. After being diluted with water, the suspension was filtered, the solid was washed with water for ten times and dried in a partial vacuum oven at 80°C for 2 days. 8.2 g of off-white polymer was obtained. ¹⁹F NMR in acetone indicated no SO₂F group.

### EXAMPLE 5

A 240-mL Shaker tube was charged with 100 mL of 1,1,2-trichlorotrifluoroethane (F113), 6 g of the CF₂=CFCF₂OCF₂CF₂SO₂F of Example 2 and 0.34 g of Perkadox™. The reaction vessel was cooled in dry ice and degassed and replaced with nitrogen gas repeatedly. 20 g of TFE and 8 g of ethylene were added into the vessel and then the tube was heated at 50°C for 10 hours. After completion of the polymerization, the unreacted monomers were removed and the white solid was washed with MeOH and acetone and dried in a partial vacuum oven at 80°C to give 23.5 g of polymer. IR(KBr) 1454 and 1472 cm⁻¹(SO₂F). DSC showed that the polymer had a broad Tm 223°C and sharp Tm 243°C. Decomposition temperature was 400°C by TGA in N₂.

A melt-pressed film of the polymer so made was immersed in a saturated LiOH solution of 1:1 water and MeOH at room temperature for 7 days. After being washed with water, the film was immersed in 1:1 water and MeOH at room temperature for 1 day, then washed with MeOH and dried in a partial vacuum oven at 80°C for 2 days.

### EXAMPLE 6

A 240-mL Shaker tube was charged with 100 mL of 1,1,2-trichlorotrifluoroethane (F113), 6 g of CF₂ = CFCF₂CF₂SO₂F of Example 1, and 0.17 g of Perkadox™. The reaction vessel was cooled in dry ice and degassed and replaced with nitrogen gas repeatedly. 12 g of vinylidene fluoride was added into the vessel and then the tube was heated at 50°C for 12 hours. After completion of the polymerization. the unreacted VF₂ was removed and the white solid was dissolved in acetone. The acetone solution was slowly poured into stirred MeOH. The solid was filtered and washed with MeOH and dried in a partial vacuum oven at 80°C to give 10.0 g of polymer. IR(KBr) 1460 (SO₂F).

### EXAMPLE 7

A 240-mL Shaker tube was charged with 100 mL of 1,1,2-trichlorotrifluoroethane (F113), 5 g of CF₂ = CFCF₂CF₂SO₂F of Example 1 and 0.17 g of Perkadox™. The reaction vessel was cooled in dry ice and degassed and replaced with nitrogen gas repeatedly. 18 g of vinylidene fluoride and 3.5 g of HFP were added into the vessel and then the tube was heated at 50°C for 10 hours and 80°C for 2 hours. After completion of the polymerization, the unreacted VF₂ and HFP were removed and white solid was dissolved in acetone. The acetone solution was slowly poured into stirred MeOH. The solid was filtered and washed with MeOH and dried in a partial vacuum oven at 80°C to give 18.4 g of polymer. IR(KBr): 1460 (SO₂F) cm⁻¹.

Mw = 1.0x10⁵ and Mn = 3.6x10⁴. The polymer had a broad Tm peak at 135°C. Decomposition temperature was 400°C in nitrogen by TGA in N₂.

### EXAMPLE 8

A suspension of 5.8 g of polymer made in Example 7, 25 mL of saturated LiOH and 30 mL of MeOH were stirred at room temperature for 36 hours. After being diluted with 200 mL of water, the suspension was filtered, the solid was washed with water and MeOH, and dried in an oven at 70°C to give 5.4 g of off-white polymer. ¹⁹F NMR indicated complete conversion of the sulfonyl fluoride group into lithium salt of sulfonic acid.

### EXAMPLES 9-11

In Examples 9-11, conductive compositions comprising hydrolyzed polymer films and organic carbonates are exemplified.

Ionic conductivity was determined using the so-called four-point probe technique described in an article entitled "Proton Conductivity of Nafion® 117 As Measured by a Four-Electrode AC Impendance Method" by Y. Sone et al., J. Electrochem. Soc., 143,1254 (1996). The method as described applies to aqueous electrolyte membranes. The method was modified for purposes of obtaining the measurements reported herein for non-aqueous solvents by placing the apparatus described in a sealed glove box purged with dry nitrogen in order to minimize any exposure to water. The method was also modified by substituting parallel linear probes traversing the full width of the test specimen for the point probes employed in the published method.

The hydrolyzed polymer was formed into films by pressing in a laboratory hydraulic press at 210°C and 3000 pounds per square inch pressure.

A 1.0 cm by 1.5 cm film was blotted dry and positioned into the conductivity cell. Cell impedance was determined over the range of 10 Hz to 100,000 Hz, and the value with zero phase angle in the higher frequency range (usually 500-5000 Hz) was ascribed to the bulk sample resistance in Ohms. The raw resistance value was then converted to conductivity, in S/cm, using the cell constant and liquid-swollen film thickness.

### EXAMPLE 9

A roughly 2" by 2" sample of the film of the hydrolyzed polymer of Example 2 was dried in a recirculating nitrogen oven (Electric Hotpack Company, Inc., Model 633, Philadelphia, PA) at 100°C for 24 hours.

The dried membrane was transferred to a sealed container while still warm and conveyed to a glove box having a positive pressure of dry nitrogen applied thereto, wherein the membrane was removed from the sealed container and allowed to come to room temperature. The membrane was cut into several sections 1.0 cm by 1.5 cm in size.

A cooled 1.0 cm by 1.5 cm membrane sample was then soaked in an excess of a 1:1 by volume mixture of ethylene carbonate (EC, Selectipur, EM Industries, Inc., Hawthorne N.Y.) and dimethyl carbonate (DMC, Selectipur, EM Industries) in a sealed glass vial for 2 hours at room temperature. The membrane was removed from the solvent bath, blotted with a paper towel to remove excess solvent, and tested using the four point probe test described above. Conductivity was 5.91x10⁻⁴ S/cm.

### EXAMPLE 10

An additional cooled 1.0 cm by 1.5 cm membrane sample from Example 9 was soaked in an excess of propylene carbonate (PC, Selectipur, EM Industries) in a sealed glass vial for 2 hours at room temperature. The membrane was removed from the solvent bath, blotted with a paper towel to remove excess solvent, and tested using the four point probe test described above. Conductivity was 1.62x 10⁻⁴ S/cm.

### EXAMPLE 11

A 1-L autoclave was charged with 300 mL of 1,1,2-trichlorotrifluoroethane (F113), 14 g of CF₂=CFCF₂OCF₂CF₂SO₂F of Example 2 and 0.30 g of Perkadox™. The reaction vessel was cooled in dry ice and degassed and replaced with nitrogen gas repeatedly. 14 g of TFE and 20 g of ethylene were added into the vessel and then the tube was slowly heated at 50°C and kept at 50°C for 10 hours. After completion of the polymerization, the unreacted monomers were removed and white suspension was obtained. After removal of solvent, the white solid was washed with MeOH and dried in a partial vacuum oven at 75°C to give 24.8 g of polymer. IR(KBr) 1454 and 1472 cm⁻¹(SO₂F). DSC showed that the polymer had a broad Tm at 175°C. Decomposition temperature was 380°C by TGA in nitrogen in N₂.

A suspension of 3.8 g of the polymer so produced and 2.5 g of LiOH in 10 mL of MeOH, 10 mL of water and 80 mL of DMSO was stirred at 80°C for 5 hours. The reaction mixture was slowly poured into a stirred 500 mL of 1:1 MeOH and water and filtered to give solid, which was washed with MeOH several times and dried in an oven at 70°C to give ionic polymer 3.3 g. ¹⁹F NMR (DMSO) indicated no sulfonyl fluoride group.

The dried hydrolyzed membrane was transferred to a sealed container while still warm and conveyed to a glove box having a positive pressure of dry nitrogen applied thereto, wherein the membrane was removed from the sealed container and allowed to come to room temperature. The membrane was cut into several sections 1.0 cm by 1.5 cm in size.

A cooled 1.0 cm by 1.5 cm membrane sample was then soaked in an excess of a 1:1 by volume mixture of EC and DMC in a sealed glass vial for 1 hour at room temperature. The membrane was removed from the solvent bath, blotted with a paper towel to remove excess solvent, and tested using the four point probe test described above. Solvent uptake was determined by the method described above. Solvent uptake was 25 %. Conductivity was 2.16x10⁻⁵ S/cm.

## Claims

1. A process for forming an ionizable composition, the process comprising: contacting, at a temperature in the range of 0-85°C, a composition comprising the functional group -(CF₂CF₂)ₙ-SO₂F, where n is 1 to 5, with a solution of an alkali metal salt, the pH of said solution not to exceed 13, for a period of time sufficient to obtain the degree of conversion desired to the alkali metal sulfonate form of the polymer.

2. The process of claim 1, wherein the composition further comprises a polymer having monomer units of vinylidene fluoride.
